Europäisches Patentamt

European Patent Office

Office européen des brevets

(19)

(11) Publication number: **0 144 764**
**B1**

(12) EUROPEAN PATENT SPECIFICATION

(45) Date of publication of patent specification: **08.06.88**

(51) Int. Cl.⁴: **A 61 B 17/22, A 61 B 17/36**

(21) Application number: **84113325.9**

(22) Date of filing: **06.11.84**

(54) **Laser catheter.**

(30) Priority: **09.12.83 US 559664**

(43) Date of publication of application:
**19.06.85 Bulletin 85/25**

(45) Publication of the grant of the patent:
**08.06.88 Bulletin 88/23**

(84) Designated Contracting States:
**AT BE CH DE FR GB IT LI NL SE**

(56) References cited:
**GB-A-2 017 506**
**US-A-4 273 109**

**LASER UND OPTOELEKTRONIK, vol. 15, no. 2,
June 1983, pages 103,104, Stuttgart, DE; K.H.
ERREY: "The gold vapour laser"**

**REVUE DE PHYSIQUE APPLIQUEE, vol. 15, no.
9, September 1980, pages 1417-1426, Paris, FR;
J.M. BRUNETAUD et al.: "Les applications
thérapeutiques des lasers"**

(73) Proprietor: **International Business Machines
Corporation
Old Orchard Road
Armonk, N.Y. 10504 (US)**

(72) Inventor: **Linsker, Ralph
372 Central Park Avenue
Scarsdale New York 10583 (US)**

(74) Representative: **Appleton, John Edward et al
IBM United Kingdom Limited Intellectual
Property Department Hursley Park
Winchester Hampshire SO21 2JN (GB)**

## Description

### Technical Field

The technical field of the invention is in the use of laser energy to remove lesions in blood vessels. In such vessels the lesion must be removed while surrounded by blood or tissue compatible fluid, without damaging the vessel to which it adheres, and without leaving toxic or otherwise injurious by-products.

### Background Art

The laser has long been looked upon as a potential source of energy for medical applications. The applications thus far in the art, however, generally use the laser either to produce desired thermal damage (e.g., retinal thermolcoagulation) or to remove tissue thermally. Thermal injury to surrounding or remaining tissue is a general feature of such laser therapy.

The laser has features that would appear to be advantageous for performing therapeutic lesion removal operations in blood vessels. The application, however, of the laser to such operations is severely limited by the conditions under which the operation is to take place.

There has been some effort in the art directed to the use of lasers in blood vessel applications. A laser catheter is shown in U.S. Patent 4,207,874 and a tip to control heat is shown in U.S. Patent 4,273,109. In the applications thus far in the art, the laser removes material pyrolytically by thermal vapourization, generally with residual charring or other thermal tissue damage. Furthermore, this vapourization method carries the risk of accidental perforation of the blood vessel wall.

Recently, it has been discovered that far ultraviolet rays in the 200 nm range, have properties that render them advantageous for use in some medical applications and such applications are shown in our pending European patent application No. 83108760.6 (Ep-A-0 111 060) which forms part of the prior art under Article 54(3) and (4) EPC only.

We believe that we are the first to discover ablative photodecomposition of a lesion in a blood vessel, in the presence of sodium chloride in solution e.g. in the blood itself, or saline solution by UV radiation of wavelength between 210 and 320 nm. The known state of the art does not include to our knowledge, the idea of applying UV radiation of wavelength between 210 and 320 nm for surgical or therapeutic purposes in the presence of sodium chloride in solution, or saline solution.

According to the invention there is provided a laser catheter arranged to deliver laser energy through an optical fibre in a catheter sheath member to ablate in vivo a lesion in a blood vessel, the sheath member being operable to exhaust the lesion destruction products, and said laser energy being in the 210 to 320 nanometer wavelength range.

In order that the invention may be fully understood it will now be described by way of example with reference to the accompanying drawings in which:-

FIG. 1 is an illustration of the laser catheter of the invention in position against a lesion in a blood vessel.

FIG. 2 is an enlarged illustration of the lesion contacting end of one embodiment of the invention.

FIG. 3 is an illustration of the lesion contacting end of another embodiment of the invention having an apertured conformal end.

FIG. 4 is an illustration of the lesion contacting end of still another embodiment of the invention having a separated passage catheter sheath.

FIG. 5 is an illustration of the lesion contacting end of still another embodiment of the invention showing an additional fibre optic for lesion visualization.

### Disclosure of the Invention

What has been discovered is a laser catheter for non-thermal ablation of lesions in a blood vessel in vivo, with little or no thermal damage to surrounding tissue.

There are several unique problems and requirements in laser removal of lesions in vivo on blood vessel walls. The lesion may be made up of organic and calcified materials each responding to different wavelength ranges for ablation or destruction. Damage to the blood vessel wall should be minimized. The risk of wall perforation should be minimized. The ablation takes place in a blood ambient or in a tissue-compatible fluid if such fluid is used to replace blood in the vicinity of the lesion during lesion removal. Blood and such replacement fluids contain concentrations of sodium chloride that strongly absorb electromagnetic energy in a wavelength range below 210 nm. and thereby reduce delivery of such energy through such a medium to a lesion. The lesion is at a location generally remote from the entry location of the catheter so that an extended length of catheter containing the energy conducting member is required, the catheter along that length being exposed to the blood and the blood vessel wall. The destruction products of the lesion, which depend on the lesion composition, may be toxic and/or particulate, and hence in general cannot be permitted to be carried away in the blood stream.

The invention provides a laser catheter with a source of electromagnetic radiation in a wavelength range that can be delivered through a sodium chloride containing medium (such as physiologic saline), that results in photochemical, non-thermal, ablation of the lesion, with little or no thermal damage to the blood vessel itself. The laser catheter can have an additional source of radiation for removal of calcified material of a lesion. The laser catheter exhausts the lesion destruction products.

It has been discovered that lesions can be removed in blood vessels by a laser catheter producing a photochemical (non-thermal) effect with little or no thermal damage to the blood vessel itself, by delivering laser energy in the 210 nanometer to 320 nanometer range to remove organic lesion material, and thereafter providing laser energy in the visible wavelength range only to the extent needed for removal of calcified material, the catheter providing means for capture and removal of both volatile and particulate lesion destruction products.

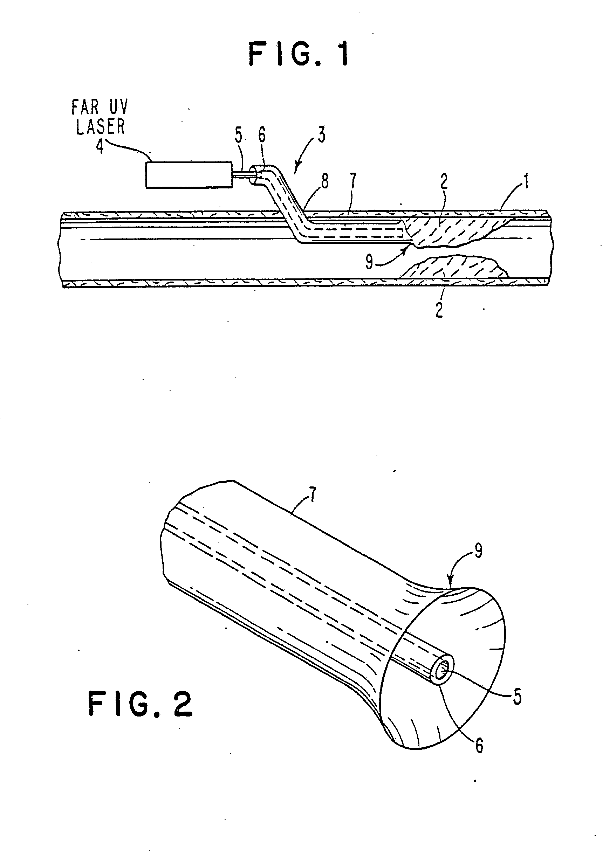

Referring to FIG. 1, in a human blood vessel 1 there is shown a lesion 2 that is interfering with the flow of blood. The laser catheter 3 of the invention is made up of a source or sources of laser energy in the 210 to 320 nanometer range, and in the above 320 nanometer range, respectively to remove organic material, and to remove calcified material. The source or sources of laser energy are shown illustratively as a single element 4, which is coupled to an optical fibre means 5 provided with a low loss cladding 6. The clad optical fibre 5, 6 in turn is positioned in a sheath 7 by any suitable means (not shown). The sheath 7 is considerably larger than the clad fibre 5, 6. The sheath 7 enters the blood vessel 1 at an opening 8 usually remote from the location of the lesion 2. The sheath 7 has a deformable ending 9 which has some resiliency and conforms to the external shape of the lesion 2 over the area of contact.

Although it was recognized and described in our previously mentioned European application that far ultraviolet energy in the 200 nanometer wavelength range would be absorbed by organic material and would break molecular bonds, it has been found in connection with the present invention that where lesion removal is in a salt-containing solution, the acceptable wavelength range is different with a lower limit of 210 nanometers and an upper limit of 320 nanometers. The 210 nanometer lower limit is just above the wavelength where sodium chloride in solution strongly absorbs energy. The 320 nm upper limit for organic tissue removal is the wavelength above which thermal damage becomes increasingly significant.

It should be noted that the molecular bond-breaking mechanism, which is responsible for organic tissue removal in the present invention, affects only a thin layer at the exposed lesion surface with each pulse of radiation. Ablation therefore proceeds to a controlled depth, there is little or no thermal damage that could affect tissue at greater depth, and the risk of accidental perforation of the vessel wall can thereby be reduced, compared with visible light laser means employing thermal vapourization.

Energy is supplied first between the limits of 210 and 320 nm for as much lesion removal as can be effected depending on lesion composition; energy in this wavelength range provides the non-thermal molecular bond-breaking mechanism which permits soft-tissue removal with little or no accompanying thermal damage. When it has been determined that all material responding to this wavelength range has been removed, and when the therapy requires further removal of calcified material, energy is delivered in the visible wavelength, and while this may produce thermal damage, the damage will be minimized, because it will only be applied to the remaining material that will not respond otherwise, and only to the extent required to remove as much as needed.

The laser energy and the clad optical fibre for the ranges of wavelength capable of destroying the different component materials that may be found in the lesion, permit energy to be delivered to the lesion 2 with high transmission efficiency, high effectiveness for lesion removal, and minimal damage to the blood vessel.

The ultraviolet energy coupled into the lesion organic components causes these lesion components to volatilize as small-molecular-weight species.

Removal of calcified material by visible laser radiation may yield particulate products; also, lesion fragments may separate from the vessel wall during ablation. Provision is made for the particulate or toxic volatile products produced in the removal of the lesion, as the energy from the laser 4 is coupled into the lesion 2, to be conducted away through the sheath 7. The sheath 7 is provided with a deformable end portion 9 which conforms to the shape of the lesion. The seal made by the portion 9 and the lesion 2 need not be very tight.

Referring next to FIG. 2, the end of the catheter is shown wherein the sheath 7 has running down its center the optical fibre 5, or fibre bundle, (depicted as a single fibre with the cladding 6), and having a deformable end 9 which can conform to the general shape of the lesion. Lesion destruction products formed as the energy is delivered through the clad optical fibre 5, 6 are removed through the region between the clad optical fibre 5, 6 and the sheath 7. If appropriate, a patient may be fitted with a blood vessel occluding device, or devices, not shown, positioned on the distal and/or proximal sides of the lesion 2, for interrupting and controlling blood flow, and for isolation of the lesion removal region from the rest of the blood stream.

The lesion 2 destruction products are carried in the sheath 7 between the clad fibre 5, 6 and the inside wall of the sheath 7. A fluid vehicle can be provided to convey the destruction products, and the end of the sheath 7 outside the blood vessel 1 can be provided with exhaust means, including a pressure means, standard in the art, to minimize leakage. Where the exhaust means results in a pressure in the sheath 7 that is less than the pressure in the blood vessel, leakage will occur from the blood vessel into the sheath, which will counter escape of destruction products into the blood stream.

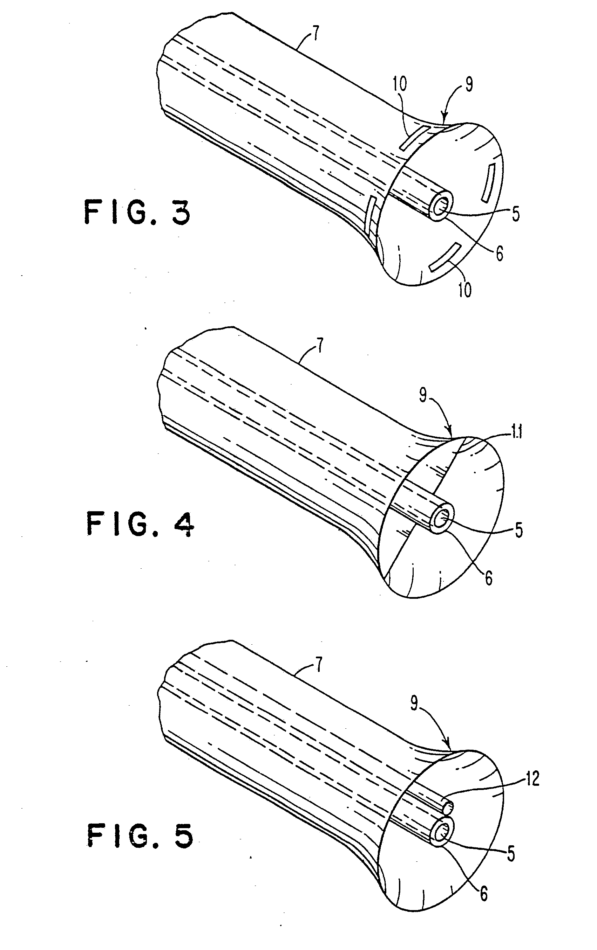

Referring next to FIG. 3, one means of destruction product removal employs the blood itself. The periphery of the deformable end 9 has openings 10 which permit a portion of the blood supply in the blood vessel 1 to enter and to carry away the lesion destruction products through the sheath 7, and outside the patient to a suitable point between the element 4 and the sheath 7 where an appropriate reservoir and pressure means (not shown) are provided.

Under situations in which blood flow at the lesion site is absent, either because the catheter and/or lesion occlude the blood vessel, or because the region is deliberately isolated by use of an occluding device, a separate supply is provided of tissue-compatible fluid, such as physiologic saline, to remove the lesion destruction products.

Referring to FIG. 4, the sheath is provided with a partitioning member 11 which supports the clad fibre 5, 6, and permits the lesion destruction products to be removed by having a fluid flow down one side of the partition across the lesion ablation interface, and back along the other side. Supply and return connections, not shown, would be provided at the end of the sheath 7 outside the patient between the end of the sheath 7 and the element 4.

In the use of a catheter, its position may be controlled by means providing direct visualization of the lesion, and/or with the aid of other apparatus e. g. a fluoroscope. In FIG. 5, within the sheath 7 is provided an optical fibre 12 which is used to aid in positioning the laser catheter adjacent and in contact or close proximity with the lesion 2.

The catheter of the invention permits laser energy, in a wavelength range providing little or no thermal damage, to be delivered by the optical fibre or bundle 5 to the site of the vascular obstruction, to ablate lesions, for example atherosclerotic lesions, in a controlled and non-thermal manner so as to avoid the risks and disadvantages of standard invasive surgical techniques. Because thermal damage to the blood vessel wall is avoided or minimized, healing may be facilitated with less risk than with methods using laser catheters to thermally vaporize tissue with visible-wavelength energy. The layer-by-layer ablation which results from using laser pulses also reduces the risk of perforating the vessel wall.

The range of wavelengths employed first ablates the organic portion of the lesion by coupling with and breaking the bonds, resulting in volatilization of the material of the lesion rather than thermally removing the lesion material, with resulting charring or other thermal damage to the blood vessel wall. Only when any remaining lesion material has significant calcification are wavelengths in the visible range used, if needed.

Best Mode For Carrying Out The Invention

Referring to FIG 1, radiation initially at 248 nanometers is provided from a Krypton-fluoride laser in the element 4. The radiation is transmitted through the optical fibre 5 provided with cladding 6. The fibre 5 is made of a high purity silica core, with cladding 6 being of an organic elastomer. This optic energy delivery combination permits highly efficient energy transmission over the required length for wavelengths in the 210 to 320 nm range and for wavelengths into the visible part of the spectrum. The laser in the element 4 is operated in a pulsed mode, providing an energy fluence of the order of 1 J/cm$^2$/pulse, which provides an ablation of about 0.3 microns thickness per pulse.

The end of the catheter is positioned using fluoroscopic means alone, or in combination with fibre optic monitoring. The deformable end 9 is positioned to conform to the shape of the lesion 2. Using the apertures 10 of FIG. 3, blood in the blood vessel 1 exhausts the lesion destruction products out through the sheath 7. When the organic portion of the lesion 2 has been removed, longer wavelengths into the visible ranges such as 0.5 micron wavelength energy from a separate argon laser in the element 4, can be delivered through the optical fibre 5.

In accordance with the invention, the longer (visible) wavelength is used only for material that remains after the shorter wavelength has accomplished essentially as much tissue removal as there is lesion material that will be ablated by it. The final ablation step can use the narrow range 210 to 320 nm wavelength, leaving a clean vascular surface with little or no thermal damage.

There will be a number of variations to the techniques described. As indicated, the optical fibre 5 maybe a single fibre or a bundled unit. One can optionally include steering means at the tip of a type currently used in gastrointestinal fibreoptic endoscopy and focusing means at the tip which would provide radiation over a well-delineated area or linear region of atheromatous plaque. When toxic or particulate products must be removed from the blood stream, suction means, not shown, is provided outside the blood vessel between the end of the sheath 7 and the element 4 for use with the sheath 7 as illustrated in FIG. 3 or 4. The simplest sheath is FIG. 3 in which blood enters the catheter through openings 10 in its tip, and carries with it any lesion destruction products generated at the site of the lesion removal. Fiber optics for visualization purposes, such as element 12 in FIG. 5, can be provided. The laser energy source(s) in element 4, can be a variable wavelength device over the range needed, or several separate wavelength laser elements.

The seal of the end 9 that deforms against the lesion need not be tight, but it should prevent any large released lesion products escaping and thereby entering the blood stream. The blood is at a small negative pressure differential in the sheath, so that it enters the catheter from the blood vessel.

The radiation in the narrow wavelength range between 210 and 320 nanometers is capable of decomposing the organic material component in blood vessel lesions by electronically exciting the constituent bonds of the material, followed by bond breaking, and the production of volatile materials which escape from the surface. This reaction is not photothermal but rather is photochemical in nature. In this photodecomposition the fragments explode from the lesion, carrying away kinetic energy.

A laser with desirable properties and operating in this range is a krypton fluoride laser with a wavelength of 248 nanometers. Typical pulse durations are about 10 nanoseconds. An argon laser for producing 0.5 micron visible light energy is well known.

What has been described is a laser catheter for non-pyrolytic ablation of lesions in vivo within blood vessels, whereby energy within a narrow ultraviolet wavelength range is delivered to the lesion to accomplish as much removal as the materials of the lesion will permit and then longer wavelength energy is delivered to the remaining material.

## Claims

1. Laser catheter arranged to deliver laser energy through an optical fibre (5) in a catheter sheath member (7) to ablate in vivo a lesion (2) in a blood vessel, the sheath member being operable to exhaust the lesion destruction products, and said laser energy being in the 210 to 320 nanometer wavelength range.

2. Laser catheter of claim 1 wherein said laser energy is initially selectable in said 210 to 320 nanometer range, and subsequently in an above 320 nanometer range.

3. Laser catheter of claim 1 wherein the sheath member is operable to exhaust said lesion destruction products by fluid flow in said sheath member.

4. Laser catheter of any preceding claim in which said sheath member has a conformal lesion-contacting resilient portion (9) at its end.

5. Laser catheter of claim 4 as dependant on claim 3 wherein said resilient portion has blood entry openings (10) therein.

6. Laser catheter of claim 3 wherein said sheath member has a dividing wall (11) for two-way fluid flow for exhausting said lesion destruction products.

7. Laser catheter of any preceding claim including optical fibre means (12) extending therethrough to aid in positioning the catheter relative to a said lesion.

8. Laser catheter of claim 2 including two lasers for providing said laser energy each of a wavelength in a respective said nanometer range.

## Patentansprüche

1. Laserkatheder, welcher so eingerichtet ist, daß er Laserenergie durch eine optische Faser (5) in einem Kathederscheidenelement (7) zur Abtragung einer Läsion (2) in einem Blutgefäß in vivo liefert, wobei das Scheidenelement so betreibbar ist, daß die Läsionszerstörungsprodukte abgezogen werden, und wobei die Laserenergie im Wellenlängenbereich von 210 bis 320 Nanometer liegt.

2. Laserkatheder nach Anspruch 1, bei welchem die Laserenergie anfänglich in dem Bereich von 210 bis 320 Nanometer und nachfolgend in einem Bereich oberhalb von 320 Nanometer auswählbar ist.

3. Laserkatheder nach Anspruch 1, bei welchem das Scheidenelement so betreibbar ist, daß die Läsionszerstörungsprodukte durch eine Fluidströmung in dem Scheidenelement abgezogen werden.

4. Laserkatheder nach irgendeinem vorstehenden Anspruch, bei welchem das Scheidenelement an seinem Ende einen formanpassenden die Läsion berührenden elastischen Abschnitt (9) aufweist.

5. Laserkatheder nach Anspruch 4 in Rückbezug auf Anspruch 3, bei welchem der elastische Abschnitt Bluteintrittsöffnungen (10) aufweist.

6. Laserkatheder nach Anspruch 3, bei welchem das Scheidenelement eine Unterteilungswand (11) für eine Zweiwegefluidströmung zum Abziehen der Läsionszerstörungsprodukte aufweist.

7. Laserkatheder nach irgendeinem vorstehenden Anspruch, welcher optische Fasermittel (12) aufweist, die sich darin zur Unterstützung beim Positionieren des Katheders relativ zur Läsion erstrecken.

8. Laserkatheder nach Anspruch 2, welcher zur Lieferung der Laserenergie jeweils einer Wellenlänge in den betreffenden Nanometerbereichen zwei Laser enthält.

## Revendications

1. Cathéter à laser agencé de manière à délivrer une énergie laser par l'intermédiaire d'une fibre optique (5) située dans un élément (7) formant enveloppe du cathéter, pour réaliser l'ablation, in vivo, d'une lèsion (2) dans un vaisseau sanguin, l'élément formant enveloppe pouvant être utilisé de manière à extraire les produits formés lors de la destruction de la lésion, et ladite énergie laser ètant située dans la gamme des longueurs d'onde allant de 210 à 320 nanomètres.

2. Cathèter à laser selon la revendication 1, dans lequel ladite énergie laser peut être initialement sélectionnée dans ladite gamme comprise entre 210 et 320 nanomètres, et ultérieurement dans une gamme supérieure à 320 nanomètres.

3. Cathéter à laser selon la revendication 1, dans lequel l'élément formant enveloppe peut être utilisé de manière à extraire lesdits produits formés lors de la destruction de la lésion grâce à la circulation d'un fluide dans ledit élément formant enveloppe.

4. Cathéter à laser selon l'une quelconque des revendications précédentes, dans lequel ledit élément formant enveloppe comporte, au niveau de son extrémité, une partie élastique (9) de forme adaptable et venant en contact avec la lésion.

5. Cathéter à laser selon la revendication 4, onsidérée comme dépendante de la revendication 3, dans lequel ladite partie èlastique comporte des orifices (10) de pénétration du sang.

6. Cathéter à laser selon la revendication 3, dans lequel ledit élément formant enveloppe comporte une paroi de subdivision (11) permettant un écoulement bidirectionnel de fluide pour l'évacuation desdits produits formés lors de la destruction de la lésion.

7. Cathéter à laser selon l'une quelconque des revendications précédentes, contenant des moyens (12) en forme de fibres oqtiques s'étendant à travers le cathéter pour faciliter le positionnement de ce dernier par rapport à ladite lésion.

8. Cathéter à laser selon la revendication 2, comportant deux lasers servant à délivrer lesdites énergies laser possédant des longueurs d'onde se situant respectivement dans ladite gamme de valeurs en nanomètres.

0 144 764

# FIG. 1

FAR UV
LASER
4

# FIG. 2

1

FIG. 3

FIG. 4

FIG. 5